**Europäisches Patentamt**

**European Patent Office**

**Office européen des brevets**

(19)

(11) Veröffentlichungsnummer: **0 224 117**
**A1**

(12) ## EUROPÄISCHE PATENTANMELDUNG

(21) Anmeldenummer: **86115632.1**

(22) Anmeldetag: **11.11.86**

(51) Int. Cl.⁴: **A 61 B 17/06**

(30) Priorität: **11.11.85 DE 3539891**

(43) Veröffentlichungstag der Anmeldung:
**03.06.87 Patentblatt 87/23**

(84) Benannte Vertragsstaaten:
**AT BE CH DE ES FR GB GR IT LI LU NL SE**

(71) Anmelder: **Hein, Wolfram**
**Auf der Wallme 34**
**D-5788 Winterberg 1(DE)**

(72) Erfinder: **Hein, Wolfram**
**Auf der Wallme 34**
**D-5788 Winterberg 1(DE)**

(74) Vertreter: **Grosse, Dietrich, Dipl.-Ing. et al,**
**Patentanwälte**
**HEMMERICH-MÜLLER-GROSSE-POLLMEIER-MEY**
**Hammerstrasse 2**
**D-5900 Siegen 1(DE)**

(54) **Chirurgische Nadel.**

(57) Eine chirurgische Nadel mit vorzugsweise gekrümmtem Nadelschaft (1), die einen Teil einer Einweg-Nadel-Faden-Kombination darstellen kann, soll in einer Anzahl möglichst feinstufig abgestufter unterschiedlicher möglicher Lagen mittels eines Nadelhalters sicher und unverdrehbar gehalten werden, wobei gleichzeitig die Gefahr des Einreißens des durchstochenen Gewebes vermieden werden soll.

Zweckmäßig ist hierfür der Nadelschaft (1) mit einem Querschnitt in Form eines gleichseitigen n-Ecks ausgeführt, wobei n als Anzahl der Ecken des Querschnittes bzw. Mantelflächen der Nadel mindestens 5 beträgt, so daß definierte Angriffsflächen in feiner Abstufung geboten werden und die zwischen Mantelflächen entlang von Kanten gebildeten Winkel sich als stumpfwinklig erweisen.

Fig.1

EP 0 224 117 A1

0224117

## Chirurgische Nadel

Die Erfindung betrifft eine chirurgische Nadel mit einem zum definierten Angreifen eines Nadelhalters prismatisch bzw. kantig ausgebildeten, vorzugsweise gekrümmten Nadelschaft, dessen vorderes Ende zu einer Nadelspitze angeschärft ist. Derartige Nadeln dienen zum Nähen von Fleischwunden.

Es sind Nadeln bekannt (US 3 394 704), die einen runden, bogenförmig gekrümmten Schaft aufweisen. An seinem vorderen Ende ist dieser Schaft angespitzt, während das hintere Ende mit einer Öffnung einen angeschlossenen Faden faßt. Eine solche Nadel wird damit als Einweg-Nadel-Faden-Kombination geliefert. Zum Einführen der Nadel in zu verbindendes Gewebe und zum Herausziehen derselben wird ein zangenähnlicher Nadelhalter verwendet, der zwei Backen aufweist, die entweder scherenartig auseinandergehen können oder parallelgeführt sind. Bei der Verwendung von Nadeln mit rundem Querschnitt ergibt sich eine Fixierung der Lage der Nadel nur durch die Pressung der Backen auf Mantellinien der Nadel. Schon bei minimalen auftretenden Momenten, die bereits durch den Arbeitsdruck bewirkt werden können, bewegt sich diese, so daß eine sichere Fixierung der Nadel im Nadelhalter nicht gewährleistet ist.

Es sind weiterhin Nadeln bekannt (GB 752 443), die einen dreieckigen Querschnitt aufweisen. Da beim Schließen des Nadelhalters eine von dessen Backen vollständig auf einer Nadelseite und die gegenüberliegende Backe auf einer Kante der Nadel aufliegen, ergibt sich eine relativ gute Fixierung der Nadel im Nadelhalter. Es sind jedoch gegenüber runden Nadeln, bei denen es beliebig viele Fixierungsmöglichkeiten gibt, nur sechs sicher fixierte Raststellungen möglich, so daß für feine Nähte sich eine zu grobe Stufung ergibt. Als nachteilig erweist sich weiterhin, daß beim dreieckigen Anschliff eines Nadelschaftes eine relativ scharfe Kante auftritt, so daß der scharfe Nadelrücken die Gefahr des Einreißens des Gewebes bedingt. Solche Einrisse treten auch auf, wenn die Kanten eines Nadelschaftes gebrochen ausgeführt sind. - Bei einem an sich möglichen quadratischen Querschnitt des Nadelschaftes ergeben sich praktisch nur vier Raststellungen, die zudem unzureichend gesichert sind, da die Linie, innerhalb deren eine der Backen ihre Kraft ausübt, nicht mittig auf der an der gegenüberliegenden Backe plan anliegenden Fläche steht, sondern vielmehr praktisch über einer von deren Seitenkanten.

Die Erfindung geht von der Aufgabe aus, eine chirurgische Nadel der eingangs genannten Art derart auszubilden, daß sich eine große Anzahl von sicher zu fixierenden Raststellungen ergibt, die eine feinstufige Einstellung der jeweiligen Lage gestatten, und die gleichzeitig relativ hohe Arbeitsdrucke aufzunehmen vermögen.

Die Aufgabe wird erfindungsgemäß dadurch gelöst, daß der Nadelschaft einen Querschnitt in Form eines gleichseitigen n-Ecks aufweist, wobei n mindestens fünf beträgt. Durch eine derartige Ausbildung ergibt sich je nach verwendetem Nadelhalter eine Vielzahl von Raststellungen bei gleichzeitigen guten Halteeigenschaften. Hierbei gilt jedoch, daß zwar, je größer n wird, um so mehr Raststellungen gegeben sind, der aufnehmbare Arbeitsdruck jedoch verringert wird. Deshalb dürfte sich als optimaler Nadelschaft ein solcher mit vorzugsweise fünfeckigem Querschnitt anbieten.

Es können beliebige Nadelhalter mit parallelen oder nicht parallelen Backen verwendet werden, wenn n ungeradzahlig ist. Der Arbeitsdruck bei der Verwendung eines Nadelhalters mit

- 3 -

parallel geführten Backen läßt sich erhöhen, wenn n geradzahlig ist.

Im einzelnen sind die Merkmale der Erfindung anhand der Beschreibung eines Ausführungsbeispieles in Verbindung mit dieses darstellenden Zeichnungen erläutert. Es zeigen hierbei:

Figur 1   eine erfindungsgemäße chirurgische Nadel,

Figur 2   einen Querschnitt eines durch Backen eines Nadelhalters erfaßten Nadelschaftes der Nadel gemäß Figur 1, und

Figur 3   einen Querschnitt durch einen sechseckigen Nadelschaft zur Einspannung in einen Nadelhalter mit parallel geführten Backen.

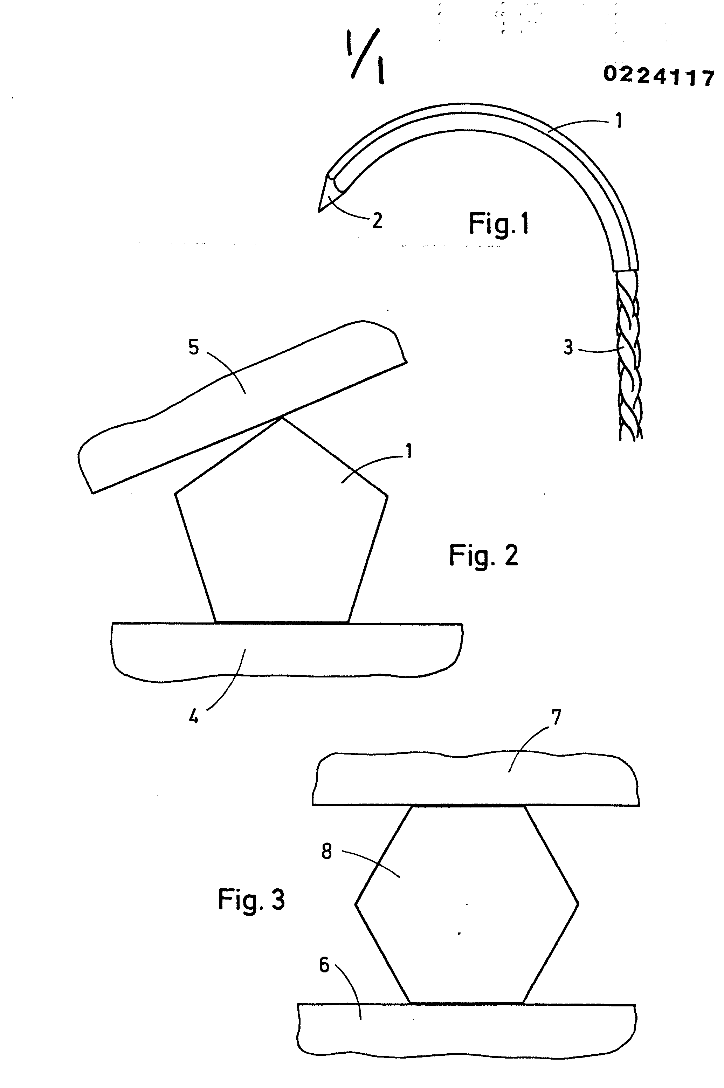

In Figur 1 ist eine chirurgische Nadel mit einem erfindungsgemäßen fünfeckigen Nadelschaft 1 dargestellt, dessen vorderes Ende als Nadelspitze 2 abgeschärft ist. Das hintere Ende des Nadelschaftes ist hohlzylindrisch ausgeführt und umfaßt das freie Ende eines Fadens 3 fest. Anhand der Figur 2 werden nunmehr die Vorteile einer derart ausgebildeten Nadel näher erläutert. Der fünfeckige Nadelschaft 1 wird von zwei Backen 4 und 5 eines Nadelhalters derart erfaßt, daß eine der Seiten des gleichseitigen Fünfecks mit seiner ganzen Länge auf der Greiffläche der Backe 4 liegt. Der Haltepunkt der jeweiligen anderen Backe 5 befindet sich, egal ob es sich um einen Nadelhalter mit scherenförmiger Backenführung oder um einen Nadelhalter mit paralleler Backenführung handelt, immer auf der der von der Backe 4 erfaßten Fläche gegenüberliegenden Kante, die auf deren Mittelsenkrechten liegt. Dadurch wird der von der Backe 5 auf die Kante ausgeübte Druck voll auf die an der Backe 4 aufliegende Fläche übertragen, ohne daß es zu einem Verkanten kommen kann. Hieraus ergibt sich ein für eine derartige Nadel relativ hoher nutzbarer Arbeitsdruck. Die fünfeckige Querschnittsform erlaubt weiterhin zehn Fixierstellungen im Nadelhalter, wobei auf jede Backe jeweils fünf Stellungen entsprechend den Seiten des Fünfecks entfallen.

Bei der Verwendung eines Querschnittes mit einer geradzahligen Eckenzahl, von der ein sechseckiger Querschnitt in Figur 3 dargestellt ist, ist zweckmäßiger Weise ein Nadelhalter mit parallelen Backen 6 und 7 einzusetzen. Hierbei ergibt sich zwar bei gleichem Durchmesser eine kleinere Seitenlänge und somit eine pro Backe 6, 7 geringere Auflagefläche. Da jedoch jeweils zwei Seiten des Nadelschaftes 1 erfaßt werden, ist insgesamt betrachtet die Auflagefläche und somit der übertragbare Arbeitsdruck größer als bei einer Nadel mit fünfeckigem Querschnitt. Dafür ergeben sich aber bei dieser Art von Nadel nur sechs gesicherte Raststellungen. Eine solche mit sechseckigem Schaft versehene Nadel läßt sich auch in Verbindung mit einem Nadelhalter mit Radialschloß, also mit scherenartiger Ausbildung, verwenden, jedoch wird hierdurch die Stabilität des Haltens in vorgegebenen Stellungen verringert, da nunmehr nur noch eine der Flächen auf einer Backe aufliegt und damit die resultierenden tragenden Flächen auf die Hälfte zurückgeführt sind. Zudem liegt die der abgestützten Fläche gegenüberliegende tragende Kante nicht mehr auf der Mittelsenkrechten dieser Fläche, sondern wird vielmehr durch die vordere obere Kante des Sechsecks gebildet und liegt auf dem Lot der vorderen unteren Kante. Da jedoch die von der Backe 7 ausgeübte Kraft auf die obere Kante schräg angreift, ergibt sich noch eine relativ sichere Haltung des Nadelschaftes 8.

Da bei den empfohlenen Nadeln der Kantenwinkel über 90° liegt ist gewährleistet, daß die jeweils tragende Kante nicht in das zu haltende Gewebe einschneidet und die durch das Nähen entstandene Perforation auszureißen vermag. Unterstützt wird dieses Verhalten, indem ihre der Krümmungsachse des Nadelschaftes zugewandte Seitenfläche desselben innerhalb einer Krümmungsachse zylindrisch umschließenden Fläche liegt, so daß in der Hauptbeanspruchungsrichtung vorstehende Kanten vermieden sind.

Über die beschriebenen Querschnittsformen der Nadeln hinaus sind noch weitere möglich, bei denen die Querschnitte regelmäßige Vielecke mit höherer Eckenzahl darstellen. Mit einer höheren Zahl der Ecken kann jedoch der Herstellungsaufwand steigen, und als positiv wirkt sich aus, daß die Zahl der möglichen Stellungen sich entsprechend erhöht, während der Widerstand gegen Drehen der Nadel zwischen den Backen des Nadelhalters abnimmt. Bei Nadelschäften mit Querschnitten eines ungeradzahligen Vielecks ergeben sich Vorzugslagen in der doppelten Anzahl der Kanten, während bei geradzahligen Vielecken die Anzahl der Vorzugsstellungen der der Ecken entspricht. In jedem dieser Fälle entstehen Nadeln, bei denen die Gefahr des Ausreißens der Einstiche in das Gewebe ebenso unterbunden ist wie eine sichere, relativ feinstufige Halterung ermöglicht wird. Die Erfindung läßt sich nicht nur auf Nadel-Faden-Kombinationen anwenden, sondern erweist sich auch bei herkömmlichen Nadeln als vorteilhaft.

Patentansprüche

1. Chirurgische Nadel mit einem zum definierten Angreifen eines Nadelhalters prismatisch bzw. kantig ausgebildeten, vorzugsweise gekrümmten Nadelschaft (1), dessen vorderes Ende zu einer Nadelspitze (2) angeschärft ist, d a d u r c h   g e k e n n z e i c h n e t , daß der Nadelschaft (1) einen Querschnitt in Form eines gleichseitigen n-Ecks aufweist, wobei n mindestens fünf beträgt.

2. Chirurgische Nadel nach Anspruch 1, d a d u r c h   g e k e n n z e i c h n e t , daß n ungeradzahlig ist.

3. Chirurgische Nadel nach Anspruch 1, d a d u r c h   g e k e n n z e i c h n e t , daß n geradzahlig ist.

4. Gekrümmte chirurgische Nadel nach einem der Ansprüche 1 bis 3, d a d u r c h   g e k e n n z e i c h n e t , daß die Innenseite der Nadel durch eine Seitenfläche des Nadelschaftes (1) gebildet ist, welche die Krümmungsachse als Zylinderabschnitt umschließt.

1/1

0224117

Fig.1

Fig. 2

Fig. 3

# EUROPÄISCHER RECHERCHENBERICHT

Europäisches Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| X | GB-A- 906 205 (AMERICAN CYANAMID COMP.) * Abbildung 7; Seite 4, Zeilen 10-18 * | 1,3 | A 61 B 17/06 |
| Y | | 2,4 | |
| | --- | | |
| Y | DE-A-1 766 521 (ETHICON) * Abbildungen; Seite 2, Zeilen 14-24 * | 2,4 | |
| | ----- | | |

| | RECHERCHIERTE SACHGEBIETE (Int. Cl.4) |
|---|---|
| | A 61 B D 05 B |

Der vorliegende Recherchenbericht wurde für alle Patentansprüche erstellt.

| Recherchenort | Abschlußdatum der Recherche | Prüfer |
|---|---|---|
| DEN HAAG | 12-03-1987 | STEENBAKKER J. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82